(19) Europäisches Patentamt
European Patent Office
Office européen des brevets

(11) Veröffentlichungsnummer : **0 346 276 B1**

(12) # EUROPÄISCHE PATENTSCHRIFT

(45) Veröffentlichungstag der Patentschrift : **09.09.92 Patentblatt 92/37**

(51) Int. Cl.$^5$ : **C07C 43/29**, C07C 205/12, C07C 69/76, C07C 25/22, C07F 7/08, C07B 37/12

(21) Anmeldenummer : **89810367.6**

(22) Anmeldetag : **18.05.89**

(54) **Substituierte Benzocyclobutene, Verfahren zu deren Herstellung und deren Verwendung.**

(30) Priorität : **27.05.88 CH 2008/88**
**19.07.88 CH 2755/88**

(43) Veröffentlichungstag der Anmeldung : **13.12.89 Patentblatt 89/50**

(45) Bekanntmachung des Hinweises auf die Patenterteilung : **09.09.92 Patentblatt 92/37**

(84) Benannte Vertragsstaaten : **CH DE FR GB IT LI NL**

(56) Entgegenhaltungen : **Keine Entgegenhaltungen**

(73) Patentinhaber : **CIBA-GEIGY AG**
**Klybeckstrasse 141**
**CH-4002 Basel (CH)**

(72) Erfinder : **Baumann, Marcus, Dr.**
**Arlesheimerstrasse 18**
**CH-4053 Basel (CH)**
Erfinder : **Fischer, Walter, Dr.**
**Vogesenstrasse 77**
**CH-4153 Reinach (CH)**
Erfinder : **Kvita, Vratislav, Dr.**
**Vogesenstrasse 71**
**CH-Reinach (CH)**
Erfinder : **Mayer, Carl W., Dr.**
**Steingrubenweg 224**
**CH-4125 Riehen (CH)**

EP 0 346 276 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft im Benzolring substituierte Benzocyclobuten-1,2-dichloride, -dibromide und -diiodide; deren Herstellung durch Umsetzung von entsprechenden 1,2-Bis(dichlor- oder dibrommethyl)benzolen mit NaI, und deren Verwendung zusammen mit Dienophilen in Diels-Alder-Reaktionen.

M.P. Cava et al beschreiben in J. Am. Chem. Soc. 72, S. 1701-1705 (1957) die Herstellung von in 1,2-Stellung bromierten und/oder iodierten Benzocyclobutenen durch die Reaktion von 1,2-Bis(dibrommethyl)benzol mit NaI in Ethanol als Lösungsmittel. Die gleiche Methode wird von J.W. Barton et al., J. Chem. Soc. Perkin Trans. 1, S.967-971 (1986) zur Herstellung von 1,2,4,5-Tetrabrombenzocyclobuten benutzt. Es werden zwar relativ gute Ausbeuten erzielt; die lange Reaktionsdauer von 48 Stunden ist jedoch nachteilig.

J.W. McOmie et al. beschreiben in Synthesis 7, S. 416-417 (1973), dass aus 1,2-Bis(dibrommethyl)-4,5-dimethoxy-benzol mit NaI in Dimethylformamid als Lösungsmittel das entsprechende Orthochinodi(brommethan) als reaktives Intermediärprodukt gebildet wird und mit Dienophilen zu Diels-Alder-Addukten reagiert.

Im Benzolring anders als mit Brom oder Chlor substituierte 1,2-Dihalogenbenzocyclobutene sind nicht bekannt.

Es wurde gefunden, dass man diese überraschend stabilen Verbindungen in guten Ausbeuten und kurzen Reaktionszeiten erhält, wenn man die Umsetzung von im Benzolring substituierten 1,2-Bis(dihalogenmethyl)-benzolen mit NaI in Gegenwart von Acetonitril durchführt.

Ein Gegenstand der Erfindung sind Verbindungen der Formel I

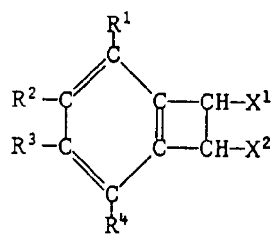

(I),

worin

$X^1$ und $X^2$ unabhängig voneinander -Cl, -Br oder -I bedeuten,

$R^1$, $R^2$, $R^3$ und $R^4$ unabhängig voneinander -F, $-CF_3$, -CN, $-NO_2$, $C_1$-$C_{18}$-Alkoxy, $C_1$-$C_{18}$-Alkylthio, $C_6$-$C_{18}$-Aryl, $C_6$-$C_{16}$-Aryloxy oder-Arylthio, Trialkylsilyl mit 3 bis 18 C-Atomen, $-COOR^5$ mit $R^5$ in der Bedeutung von H oder eines um eine Hydroxylgruppe verminderten Restes eines $C_1$-$C_{18}$-Alkoholes, oder -COX, worin X $-NH_2$ oder den einwertigen Rest eines primären oder sekundären Amins mit 1 bis 18 C-Atomen darstellt, bedeuten, und 1 bis 3 der Gruppen $R^1$ bis $R^4$ unabhängig voneinander H, -Cl oder -Br sind und die vierte der Gruppen $R^1$ bis $R^4$ eine der zuvor angegebenen Bedeutungen hat.

Vorzugsweise stehen $X^1$ und $X^2$ entweder für -Br oder für -I und vorzugsweise bedeuten $R^1$, oder $R^1$ und $R^4$, oder $R^1$, $R^2$ und $R^4$ ein Wasserstoffatom.

$R^1$-$R^4$ als Alkoxy und Alkylthio können linear oder verzweigt sein und enthalten bevorzugt 1 bis 12, besonders 1 bis 6 und insbesondere 1 bis 4 C-Atome. Beispiele sind Alkoxy- und Alkylthioreste von Methyl, Ethyl, die Isomeren von Propyl, Butyl, Pentyl, Hexyl, Heptyl, Octyl, Nonyl, Decyl, Undecyl, Dodecyl, Tridecyl, Tetradecyl, Pentadecyl, Heptadecyl und Octadecyl.

Stellen $R^1$-$R^4$ Aryl oder einen Aryl enthaltenden Rest dar, so ist das Aryl z.B. Naphthyl oder besonders Phenyl. In einer bevorzugten Untergruppe stellen $R^1$-$R^4$ in der Bedeutung von Aryl oder einem Aryl enthaltenden Rest Phenyl, Phenyloxy oder Phenylthio dar.

$R^1$-$R^4$ enthalten als Trialkylsilyl bevorzugt 3 bis 14, besonders 3 bis 8 C-Atome. Beispiele für Alkylgruppen sind zuvor erwähnt worden. Einige Beispiele sind Trimethyl-, Triethyl-, Dimethylethyl-, Tributyl-und Dimethylbutylsilyl.

$R^5$ als Rest eines Alkohols enthält bevorzugt 1-12, besonders 1 bis 6 C-Atome. $R^5$ kann z.B. lineares oder verzweigtes Alkyl, gegebenenfalls mit $C_1$-$C_6$-Alkyl substituiertes $C_5$- oder $C_6$-Cycloalkyl, oder gegebenenfalls mit $C_1$-$C_6$-Alkyl substituiertes $C_6$-$C_{12}$-Aryl oder $C_6$-$C_{12}$-Aryl-$C_xH_{2x}$ mit x gleich 1 bis 4 sein. Aryl ist bevorzugt Phenyl und x ist bevorzugt 1. Bevorzugt stellt $R^5$ H, $C_1$-$C_{18}$-, besonders $C_1$-$C_{12}$- und besonders $C_1$-$C_6$-Alkyl, Cyclohexyl, Cyclopentyl, Phenyl oder Benzyl dar. Einige Beispiele sind Methyl, Ethyl, die Isomeren von Propyl, Butyl, Pentyl, Hexyl, Heptyl, Octyl, Nonyl, Decyl, Undecyl, Dodecyl, Tetradecyl, Hexadecyl, Octadecyl, Cyclopentyl, Cyclohexyl, Methylcyclohexyl, Phenyl, Benzyl, Methylphenyl, Dimethylphenyl, Ethylphenyl, Methylethylphenyl, t-Butylphenyl und Methylbenzyl. Besonders bevorzugt stellt $R^5$ Methyl oder Ethyl dar.

X enthält in der Bedeutung von primärem oder sekundärem Amino bevorzugt 1 bis 18, besonders 1 bis 12 und insbesondere 1 bis 8 C-Atome. X kann der Formel $-NR^6R^7$ entsprechen, worin $R^6$ und $R^7$ unabhängig von-

einander H, lineares oder verzweigtes $C_1$-$C_{18}$-, besonders $C_1$-$C_{12}$- und insbesondere $C_1$-$C_6$-Alkyl, Cyclopentyl, Cyclohexyl, Phenyl, Benzyl, oder $R^6$ und $R^7$ zusammen Tetra- oder Pentamethylen oder 3-Oxa-1,5-pentylen bedeuten. Einige Beispiele sind Dimethyl-, Diethyl-, Ethylmethyl-, Dibutyl-, Dioctyl-, Methyldodecyl-, Didodecyl-, Methyloctadecyl-, Phenylmethyl-, Benzylmethylamino, Pyrrolino, Piperidino und Morpholino.

In einer bevorzugten Gruppe von Verbindungen der Formel I sind ein oder zwei der Gruppen $R^1$ bis $R^4$ -Cl oder -Br. In einer anderen bevorzugten Gruppe sind $R^2$ -Cl oder -Br, $R^1$ und $R^4$ H und $R^3$ hat die zuvor angegebenen Bedeutungen. Eine bevorzugte Ausführungsform von Verbindungen der Formel I sind solche, worin $R^1$, $R^2$, $R^3$ und $R^4$ -F, $-CF_3$, -CN, $-NO_2$, $C_1$-$C_{12}$-Alkoxy, $C_1$-$C_{12}$-Alkylthio, $Si(CH_3)_3$, $COOR^5$ mit $R^5$ in der Bedeutung von $C_1$-$C_{18}$-Alkyl, oder -COX darstellen, wobei X $-NR^6R^7$ entspricht, worin $R^6$ und $R^7$ unabhängig voneinander H, $C_1$-$C_{18}$-Alkyl, Cyclohexyl, Cyclopentyl, Phenyl oder Benzyl oder $R^6$ und $R^7$ zusammen Tetra-oder Pentamethylen oder 3-Oxa-1,5-pentylen bedeuten, und 1 bis 3 der Gruppen $R^1$, $R^2$, $R^3$ und $R^4$ unabhängig voneinander H, -Cl oder -Br sind und die vierte der Gruppen $R^1$, $R^2$, $R^3$ und $R^4$ die zuvor angegebene Bedeutung hat. Eine Untergruppe der bevorzugten Ausführungsform sind solche Verbindungen der Formel I, worin $R^2$, $R^3$ und $R^4$ -F, $-CF_3$, -CN, $-NO_2$, $C_1$-$C_6$-Alkoxy, $C_1$-$C_6$-Alkylthio, $-Si(CH_3)_3$, $-COOC_1$-$C_{12}$-Alkyl, $-CONR^6R^7$ mit $R^6$ und $R^7$ unabhängig voneinander in der Bedeutung von H oder $C_1$-$C_{12}$-Alkyl darstellen, und $R^1$ oder $R^1$ und $R^4$ H bedeuten.

Ein weiterer Gegenstand der Erfindung ist ein Verfahren zur Herstellung von Verbindungen der Formel I, dadurch gekennzeichnet, dass man eine Verbindung der Formel II

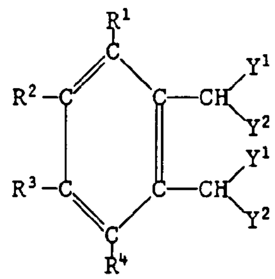

(II),

worin $R^1$, $R^2$, $R^3$, und $R^4$ die zuvor angegebenen Bedeutungen haben und $Y^1$ und $Y^2$ unabhängig voneinander für -Cl oder -Br stehen, in Gegenwart von Acetonitril oder Aceton bei einer Temperatur von mindestens 40°C mit NaI umsetzt.

$Y^1$ und $Y^2$ stehen bevorzugt entweder für -Cl oder für -Br. Die Reaktion wird vorteilhaft unter Rückflussbedingungen durchgeführt. Das Acetonitril wird zweckmässig als Lösungsmittel verwendet. Das NaI wird im allgemeinen im Ueberschuss zugegeben. Bevorzugt werden mindestens 1 Mol bis zu einem zehnfachen molaren Ueberschuss NaI verwendet, bezogen auf die Verbindungen der Formel II.

Die Isolierung der Verbindungen der Formel I kann in allgemein bekannter Weise erfolgen, z.B. Entfernung des Acetonitrils im Vakuum, Aufnahme des Rückstandes in einem organischen Lösungsmittel, Waschen mit einem Reduktionsmittel zur Entfernung von gebildetem Iod, Trocknen und Entfernen des Lösungsmittels. Der Rückstand kann in üblicher Weise durch Destillation, Kristallisation oder chromatographische Methoden gereinigt werden.

Bei der Reaktion kann ein Austausch von -Cl oder -Br durch -I stattfinden, so dass Verbindungen der Formel I mit $X^1$ und $X^2$ gleich -Cl oder -Br solche Verbindungen mit $X^1$ gleich -Cl oder -Br und $X^2$ gleich -I oder $X^1$ und $X^2$ gleich I enthalten können. Diese Gemische werden von der Erfindung umfasst.

Die Verbindungen der Formel II sind teilweise bekannt oder können nach bekannten und analogen Verfahren hergestellt werden, z.B. durch Chlorierung bzw. Bromierung von entsprechend substituierten o-Xylolen oder Phthalaldehyden. Substituierte o-Xylole und Phthalaldehyde sind nach allgemein bekannten Methoden erhältlich.

Die Verbindungen der Formel I sind wertvolle Dienkomponenten, die zusammen mit Dienophilen in hohen Ausbeuten Diels-Alder-Addukte bilden. Ein weiterer Gegenstand der Erfindung ist die Verwendung von Verbindungen der Formel I als Dienkomponente zusammen mit einem Dienophil, besonders unsubstituierten oder substituierten Benzo-1,4-chinonen oder Naphtho-1,4-chinonen, Maleinsäureanhydrid oder -imid, in Diels-Alder-Reaktionen. Hierbei erhält man substituierte Anthra-1,4-chinone, Naphthacen-5,12-chinone, oder Naphthalin-2,3-dicarbonsäureanhydride oder -imide. Anthrachinone und Naphthacenchinone können z.B. als Photosensibilisatoren (vgl. US-PS 3,941,759) oder Photoinitiatoren verwendet werden, besonders solche Naphthacen-5,12-chinone, die mindestens in 2-Stellung mit $C_1$-$C_{18}$-Alkylthio oder Phenylthio substituiert sind. Anthrachinone können auch in elektrochromen Displays verwendet werden (JP-OS 61-43680).

Die Verbindungen der Formel I bzw. daraus herstellbare Chinone sind wertvolle Zwischenprodukte zur Herstellung von substituierten Tetrathio bzw. Tetraselenotetracenen (vgl. US-PS 4 617 151). Aus solchen chalkogenierten Tetracenen können mit Elektronenacceptoren elektrisch leitende Charge-Transferkomplexe (CT-Komplexe) hergestellt werden. Mit deren funktionellen Gruppen kann man sie an Polymere binden, z.B. als Seitengruppen in Polymere einbauen kann (vgl. US-PS 4,617,151). Die CT-Komplexe eignen sich auch zur Herstellung z.B. von antistatischen Beschichtungen photographischer Filmelemente, Magnetbänder, elektrophotographischer Filmelemente und elektronischer Komponenten (siehe US-PS 3,634,336). Die chalkogenierten Tetracene weisen ferner elektrochrome Eigenschaften auf; sie können für elektrochrome Displays verwendet werden. Sie eignen sich auch als laser-optische Datenspeicher [Nach. Chem. Techn. Lab. 35, S. 255 ff (1987)] und als Anodenmaterial in organischen Solid-State-Batterien (EP-A-0 090 598). CT-Komplexe von substituierten Tetrathio-oder Tetraselenotetracenen können zur Erzielung antistatischer Eigenschaften auch in thermoplastische, duroplastische oder elastomere Polymere eingearbeitet werden. Hierzu werden zweckmässig z.B. substituierte oder unsubstituierte Tetrathio- bzw. Tetraselenotetracene zusammen mit einem löslichen Polymer oder einer Vorstufe davon und einem Elektronenacceptor, z.B. einem Halogen bildenden Mittel (organische halogenierte Verbindungen wie z.B. Bromoform, Trichlorbrommethan, Tetrabrommethan, Hexachlorpropan, Perchlorbutadien, 1,3-oder 1,4-Dichlor-2-buten, 1,4-Bis(trichloromethyl)-benzol, Iodacetonitril, Iodoform, Tetrachlorethylen, Perchlorcyclobutadien, N-Chlor-, -brom oder -iodsuccinimid) gegebenenfalls zusammen mit einem weiteren inerten Lösungsmittel gelöst und das überschüssige Halogen bildende Mittel und das Lösungsmittel bei höherer Temperatur verdampft. Die gebildete Zusammensetzung enthält im Polymer ein Netzwerk nadelförmiger Kristalle des CT-Komplexes, wenn das chalkogenierte Tetracen unsubstituiert ist oder kleine Substituenten (z.B. F, $CH_3$, $CF_3$) enthält. Solche Zusammensetzungen weisen eine hohe elektrische Leitfähigkeit auf. Diese kann noch verbessert werden, wenn man ein aus den Verbindungen der Formel I hergestelltes substituiertes Tetrathio-oder Tetraselenotetracen mitverwendet, das kein solches Netzwerk bildet und das in der Polymermatrix in feiner Verteilung vorliegt, da solche substituierten Tetrathio- oder Tetraselenotetracene keine oder nur eine geringe Kristallisationstendenz im Polymer besitzen.

Die nachfolgenden Beispiele erläutern die Erfindung näher.

A) Herstellungsbeispiele

Beispiele 1-7:

Eine Mischung aus 0,03 Mol entsprechend substituiertem 1,2-Bis(dibrommethyl)-benzol, 0,18 Mol Natriumiodid und 100 ml Acetonitril wird unter Rühren 1-2 Stunden am Rückfluss erhitzt. Nach dem Abkühlen wird mehrmals mit Hexan extrahiert und die Hexanphase mit $NaHSO_3$-Lösung gewaschen. Nach dem Abdampfen des Hexans wird der Rückstand durch Destillation oder Kristallisation bzw. Chromatographie gereinigt. Auf diese Weise werden die in Tabelle 1 zusammgestellten Gemische an substituierten 1,2-Dibrom- bzw. 1,2-Bromiodbenzocyclobutenen erhalten.

Tabelle 1

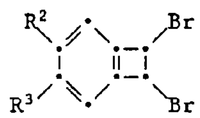 (A); 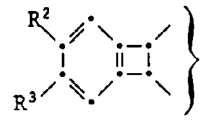 Br, I (B)

| Beispiel | $R^2$ | $R^3$ | Ausbeute % | Siedepunkt/ Schmelzpunkt | Verbindung A(%) | Verbindung B(%) | Massenspektrum $M_A^{\oplus}$/% | Massenspektrum $M_B^{\oplus}$/% | Bemerkungen |
|---|---|---|---|---|---|---|---|---|---|
| 1 | H | $OCH_3$ | 43 | 150°C 0,013 mbar | 5 | 95 | 292/0,3 | 338/1 | a) |
| 2 | H | $NO_2$ | 19 | 112-113°C | 5 | 95 | 307/0,1 | 353/4 | b) |
| 3 | H | $COOCH_3$ | 63 | - | 55 | 45 | 320/42 | 366/0,1 | c) |
| 4 | H | F | 42 | 100°C 0,052 mbar | 55 | 45 | 280/4 | 326/2 | d) |
| 5 | H | $Me_3Si$ | 80 | - | 58 | 42 | 334/42 | - | c) |
| 6 | $Me_3Si$ | $Me_3Si$ | 7 | - | 66 | 33 | 406/23 | 570/6 | e) |
| 7 | $COOCH_3$ | $COOCH_3$ | 41 | 69-71°C | 53 | 47 | 378/60 | - | f) |
| 8 | $CF_3$ | H | 63 | - | 55 | 45 | 330/10 | 377/4 | c) |
| 9 | $CF_3$ | $CF_3$ | 45 | - | 56 | 44 | 398/35 | 444/8 | c) |

a) Nach dem Abkühlen wird das Reaktionsgemisch zur Trockne eingedampft, der Rückstand zwischen Ether und Wasser verteilt und die Etherphase mit $NaHSO_3$-Lösung gewaschen. Nach dem Abdampfen des Ethers wird der Rückstand im Hochvakuum destilliert.
b) Es wird gleich verfahren wie unter a). Der Rückstand wird an Kieselgel chromatographiert (Hexan + 0,5 % Essigester) und anschliessend aus Cyclohexan kristallisiert.
c) Es wird gleich verfahren wie unter a). Der Rückstand wird an Kiesegel chromatographiert (Hexan + 0,5 % Essigester).
d) Reinigung durch Destillation
e) Chromatographie des Rückstandes in Hexan an kieselgel.
f) Chromatographie des Rücktandes mit $CH_2Cl_2$ an Kieselgel und Um-kristallisation aus Methanol.

B) Anwendungsbeispiele

Beispiele 10-11:

Eine Mischung aus 1 Mol substituiertem 1,2-Dibrom- und 1,2-Bromiod-benzocyclobuten, 1,5 Mol Naphthochinon und 7 l Dichlorbenzol wird am Rückfluss erhitzt. Nach dem Abkühlen wird das ausgefallene Anthracen-5,12-chinon abfiltriert und durch Kristallisation bzw. Sublimation im Hochvakuum gereinigt. Weitere Angaben sind in Tabelle 2 zu finden.

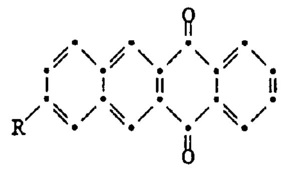

| Beispiel | R | Reaktionszeit (h) | Reinigung | Ausbeute (%) | Schmelzpunkt (°C) | Massenspektrum ($M^+$/%) |
|---|---|---|---|---|---|---|
| 10 | $-COOCH_3$ | 16 | Umkristallisation aus Toluol | 10 | 268 | 316/100 |
| 11 | $-NO_2$ | 4 | Sublimation bei 200°C | 22 | >270 | 303/100 |

Beispiel 12: (Herstellung eines elektrisch leitenden Polymerfilms)

Die Reaktion von Naphtho-1,4-chinon mit der Verbindung von Beispiel 4 ergibt 9-Fluor-Naphthacen-5,12-chinon, das nach dem in der US-PS 4,522,754, Beispiele c) bis e) beschriebenen Verfahren in 2-Fluor-5,6,11,12-tetraselenotetracen übergeführt wird.

100 mg Polyethersulfon $[\{OC_6H_4-C(CH_3)_2-C_6H_4-O-C_6H_4-SO_2-C_6H_4\}]$ und 1,6 mg 2-Fluor-5,6,11,12-tetraselenotetracen werden in 6,0 g Dimethylformamid gelöst. Dann vermischt man mit einer Lösung von 4,2 mg Hexachlorpropan in 2 ml Dimethylformamid. Die Lösung wird auf eine beheizte Glasplatte gegossen und das Lösungsmittel bei 120°C verdampft. Der spezifische Widerstand des gebildeten Polymerfilms beträgt 0,56 Ohm x cm.

## Patentansprüche

1. Verbindungen der Formel I

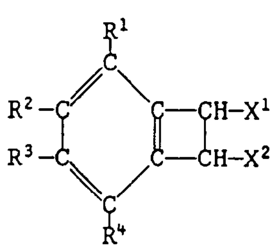

(I),

worin
$X^1$ und $X^2$ unabhängig voneinander -Cl, -Br oder -I bedeuten,
$R^1$, $R^2$, $R^3$ und $R^4$ unabhängig voneinander -F, $-CF_3$, -CN, $-NO_2$, $C_1$-$C_{18}$-Alkoxy, $C_1$-$C_{18}$-Alkylthio, $C_6$-$C_{18}$-Aryl, $C_6$-$C_{16}$-Aryloxy oder-Arylthio, Trialkylsilyl mit 3 bis 18 C-Atomen, $-COOR^5$ mit $R^5$ in der Bedeutung von H oder eines um eine Hydroxylgruppe verminderten Restes eines $C_1$-$C_{18}$-Alkoholes, oder -COX, worin X $-NH_2$ oder den einwertigen Rest eines primären oder sekundären Amins mit 1 bis 18 C-Atomen darstellt, bedeuten, und 1 bis 3 der Gruppen $R^1$ bis $R^4$ unabhängig voneinander H, -Cl oder -Br sind und die vierte der Gruppen $R^1$ bis $R^4$ eine der zuvor angegebenen Bedeutungen hat.

2. Verbindungen gemäss Anspruch 1, worin $X^1$ und $X^2$ -Br oder -I bedeuten.

3. Verbindungen gemäss Anspruch 1, worin $R^1$, oder $R^1$ und $R^4$ für H stehen.

4. Verbindungen gemäss Anspruch 1, worin $R^1$, $R^2$ und $R^4$ für H stehen.

5. Verbindungen gemäss Anspruch 1, worin $R^1$, $R^2$, $R^3$ und $R^4$ in der Bedeutung von Alkoxy und Alkylthio 1 bis 12 C-Atome enthalten.

6. Verbindungen gemäss Anspruch 1, worin $R^1$, $R^2$, $R^3$ und $R^4$ als Aryl oder einen Aryl enthaltenden Rest Phenyl, Phenyloxy oder Phenylthio darstellen.

7. Verbindungen gemäss Anspruch 1, worin $R^1$, $R^2$, $R^3$ und $R^4$ als Trialkylsilyl 3 bis 14 C-Atome enthalten.

8. Verbindungen gemäss Anspruch 1, worin $R^5$ H, $C_1$-$C_{18}$-Alkyl, Cyclohexyl, Cyclopentyl, Phenyl oder Benzyl ist.

9. Verbindungen gemäss Anspruch 1, worin X der Formel -$NR^6R^7$ entspricht, worin $R^6$ und $R^7$ unabhängig voneinander H, $C_1$-$C_{18}$-Alkyl, Cyclohexyl, Cyclopentyl, Phenyl oder Benzyl oder $R^6$ und $R^7$ zusammen Tetra- oder Pentamethylen oder 3-Oxa-1,5-pentylen bedeuten

10. Verbindungen gemäss Anspruch 1, worin eine oder zwei Gruppen $R^1$, $R^2$, $R^3$ und $R^4$ -Cl oder -Br sind.

11. Verbindungen gemäss Anspruch 1, worin $R^2$ -Cl oder -Br ist, $R^1$ und $R^4$ H sind und $R^3$ die in Anspruch 1 angegebene Bedeutung hat.

12. Verbindungen gemäss Anspruch 1, worin $R^1$, $R^2$, $R^3$ und $R^4$ -F, -$CF_3$, -CN, -$NO_2$, $C_1$-$C_{12}$-Alkoxy, $C_1$-$C_{12}$-Alkylthio, $Si(CH_3)_3$, -$COOR^5$ mit $R^5$ in der Bedeutung von $C_1$-$C_{18}$-Alkyl, oder -COX darstellen, wobei X -$NR^6R^7$ entspricht, worin $R^6$ und $R^7$ unabhängig voneinander H, $C_1$-$C_{18}$-Alkyl, Cyclohexyl, Cyclopentyl, Phenyl oder Benzyl oder $R^6$ und $R^7$ zusammen Tetra-oder Pentamethylen oder 3-Oxa-1,5-pentylen bedeuten, und 1 bis 3 der Gruppen $R^1$, $R^2$, $R^3$ und $R^4$ unabhängig voneinander H, Cl oder Br sind und die vierte der Gruppen $R^1$, $R^2$, $R^3$ und $R^4$ die zuvor angegebene Bedeutung hat.

13. Verbindungen gemäss Anspruch 12, worin $R^2$, $R^3$ und $R^4$ -F, -$CF_3$, -CN, -$NO_2$, $C_1$-$C_6$-Alkoxy, $C_1$-$C_6$-Alkylthio, -$Si(CH_3)_3$, -$COOC_1$-$C_{12}$-Alkyl, -$CONR^6R^7$ mit $R^6$ und $R^7$ unabhängig voneinander in der Bedeutung von H oder $C_1$-$C_{12}$-Alkyl darstellen, und $R^1$ oder $R^1$ und $R^4$ H bedeuten.

14. Verfahren zur Herstellung von Verbindungen der Formel I gemäss Anspruch 1, dadurch gekennzeichnet, dass man eine Verbindung der Formel II

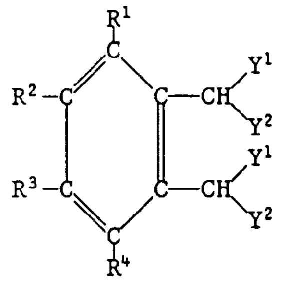

(II),

worin $R^1$, $R^2$, $R^3$ und $R^4$ die in Anspruch 1 angegebenen Bedeutungen haben und $Y^1$ und $Y^2$ unabhängig voneinander für -Cl oder -Br stehen, in Gegenwart von Acetonitril oder Aceton bei einer Temperatur von mindestens 40°C mit NaI umsetzt.

15. Verfahren gemäss Anspruch 14, dadurch gekennzeichnet, dass $Y^1$ und $Y^2$ in Formel I entweder für -Cl oder für -Br stehen.

16. Verfahren gemäss Anspruch 14, dadurch gekennzeichnet, dass die Reaktion unter Rückflussbedingungen durchgeführt wird.

**17.** Verfahren gemäss Anspruch 14, dadurch gekennzeichnet, dass mindestens 1 Mol bis zu einem zehnfachen molaren Ueberschuss NaI verwendet wird, bezogen auf die Verbindung der Formel II.

**18.** Verfahren gemäss Anspruch 14, dadurch gekennzeichnet, dass Acetonitril als Lösungsmittel verwendet wird.

**19.** Verwendung von Verbindungen der Formel I zusammen mit einem Dienophil in Diels-Alder-Reaktionen.

**20.** Verwendung gemäss Anspruch 18, worin das Dienophil ein unsubstituiertes oder substituiertes Benzo-1,4-chinon oder Naphtho-1,4-chinon ist.

## Claims

**1.** A compound of the formula I

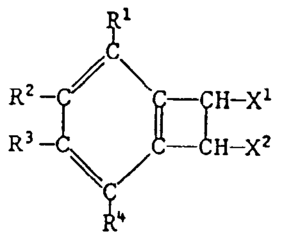

(I),

in which $X^1$ and $X^2$ independently of one another are -Cl, -Br or -I, and $R^1$, $R^2$, $R^3$ and $R^4$ independently of one another are -F, $-CF_3$, -CN, $-NO_2$, $C_1$-$C_{18}$alkoxy, $C_1$-$C_{18}$alkylthio, $C_6$-$C_{18}$aryl, $C_6$-$C_{16}$aryloxy or $C_6$-$C_{16}$arylthio, trialkylsilyl having 3 to 18 C atoms, $-COOR^5$ in which $R^5$ is H, or a radical diminished by a hydroxyl group of a $C_1$-$C_{18}$ alcohol, or -COX in which X is $-NH_2$ or the monovalent radical of a primary or secondary amine having 1 to 18 C atoms, and 1 to 3 of the groups $R^1$ to $R^4$ independently of one another are H, -Cl or -Br and the fourth of the groups $R^1$ to $R^4$ is as defined above.

**2.** A compound according to claim 1, in which $X^1$ and $X^2$ are -Br or -I.

**3.** A compound according to claim 1, in which $R^1$, or $R^1$ and $R^4$ is/are H.

**4.** A compound according to claim 1, in which $R^1$, $R^2$ and $R^4$ are H.

**5.** A compound according to claim 1, in which $R^1$, $R^2$, $R^3$ and $R^4$, as alkoxy and alkylthio, contain 1 to 12 C atoms.

**6.** A compound according to claim 1, in which $R^1$, $R^2$, $R^3$ and $R^4$, as aryl or as a radical containing aryl, are phenyl, phenoxy or phenylthio.

**7.** A compound according to claim 1, in which $R^1$, $R^2$, $R^3$ and $R^4$, as trialkylsilyl, contain 3 to 14 C atoms.

**8.** A compound according to claim 1, in which $R^5$ is H, $C_1$-$C_{18}$alkyl, cyclohexyl, cyclopentyl, phenyl or benzyl.

**9.** A compound according to claim 1, in which X has the formula $-NR^6R^7$ in which $R^6$ and $R^7$ independently of one another are H, $C_1$-$C_{18}$alkyl, cyclohexyl, cyclopentyl, phenyl or benzyl, or $R^6$ and $R^7$ together are tetramethylene, pentamethylene or 3-oxa-1,5-pentylene.

**10.** A compound according to claim 1, in which one or two groups $R^1$, $R^2$, $R^3$ and $R^4$ are -Cl or -Br.

**11.** A compound according to claim 1, in which $R^2$ is -Cl or -Br, $R^1$ and $R^4$ are H and $R^3$ is as defined in claim 1.

**12.** A compound according to claim 1, in which $R^1$, $R^2$, $R^3$ and $R^4$ are -F, - $CF_3$, -CN, -$NO_2$, $C_1$-$C_{12}$alkoxy, $C_1$-$C_{12}$alkylthio, $Si(CH_3)_3$, -$COOR^5$ in which $R^5$ is $C_1$-$C_{18}$alkyl, or -COX in which X is -$NR^6R^7$ in which $R^6$ and $R^7$ independently of one another are H, $C_1$-$C_{18}$alkyl, cyclohexyl, cyclopentyl, phenyl or benzyl, or $R^6$ and $R^7$ together are tetramethylene, pentamethylene or 3-oxa-1,5-pentylene, and 1 to 3 of the groups $R^1$, $R^2$, $R^3$ and $R^4$ independently of one another are H, Cl or Br and the fourth of the groups $R^1$, $R^2$, $R^3$ and $R^4$ is as defined above.

**13.** A compound according to claim 12, in which $R^2$, $R^3$ and $R^4$ are - F, - $CF_3$, - CN, -$NO_2$, $C_1$-$C_6$alkoxy, $C_1$-$C_6$alkylthio, -$Si(CH_3)_3$, -$COOC_1$-$C_{12}$alkyl or -$CONR^6R^7$ in which $R^6$ and $R^7$ independently of one another are H or $C_1$-$C_{12}$alkyl, and $R^1$, or $R^1$ and $R^4$ is/are H.

**14.** A process for the preparation of a compound of the formula I according to claim 1, which comprises reacting a compound of the formula II

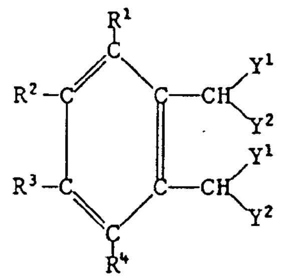

(II),

in which $R^1$, $R^2$, $R^3$ and $R^4$ are as defined in claim 1 and $Y^1$ and $Y^2$ independently of one another are -C1 or -Br, with NaI in the presence of acetonitrile or acetone at a temperature of at least 40°C.

**15.** A process according to claim 14, wherein $Y^1$ and $Y^2$ in formula I are either -Cl or -Br.

**16.** A process according to claim 14, wherein the reaction is carried out under reflux conditions.

**17.** A process according to claim 14, wherein at least 1 mol up to a ten-fold molar excess of NaI is used, relative to the compound of the formula II.

**18.** A process according to claim 14, wherein acetonitrile is used as the solvent.

**19.** The use of a compound of the formula I together with a dienophile in Diels-Alder reactions.

**20.** The use according to claim 18, wherein the dienophile is an unsubstituted or substituted benzo-1,4-quinone or naphtho-1,4-quinone.

## Revendications

**1.** Les composés de Formule I ci-dessous

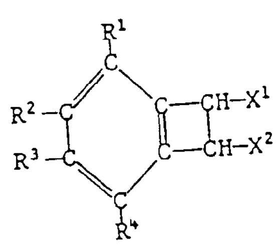

(I),

dans laquelle
$X^1$ et $X^2$ représentent chacun, indépendamment l'un de l'autre, -Cl, -Br ou -I,
$R^1$, $R^2$, $R^3$ et $R^4$ représentent chacun, indépendamment les uns des autres, -F, -$CF_3$, -CN, -$NO_2$, un $C_1$-

$C_{18}$-alcoxy, un $C_1$-$C_{18}$-alkylthio, un $C_6$-$C_{18}$-aryle, un $C_6$-$C_{16}$-aryloxy ou-arylthio, un trialkylsilyle ayant de 3 à 18 atomes de carbone ou encore un groupe $-COOR^5$, $R^5$ étant l'hydrogène ou le radical d'un alcool en $C_1$-$C_{18}$ ayant perdu un hydroxyle, ou bien un groupe -COX, X désignant le radical $-NH_2$ ou le radical monovalent d'une amine primaire ou secondaire ayant de 1 à 18 atomes de carbone, et de 1 à 3 des radicaux $R^1$ à $R^4$ sont indépendamment l'hydrogène, le chlore ou le brome, le ou les autres ayant l'une des significations indiquées ci-dessus

2. Composés selon la revendication 1 dans lesquels $X^1$ et $X^2$ sont le brome ou l'iode.

3. Composés selon la revendication 1 dans lesquels $R^1$, ou bien $R^1$ et $R^4$, sont l'hydrogène.

4. Composés selon la revendication 1 dans lesquels $R^1$, $R^2$ et $R^4$ sont l'hydrogène.

5. Composés selon la revendication 1 dans lesquels $R^1$, $R^2$, $R^3$ et $R^4$, comme groupes alcoxy ou alkylthio, ont de 1 à 12 atomes de carbone.

6. Composés selon la revendication 1 dans lesquels $R^1$, $R^2$, $R^3$ et $R^4$, comme aryles ou radicaux avec des aryles sont des groupes phényle, phényloxy ou phénylthio.

7. Composés selon la revendication 1 dans lesquels $R^1$, $R^2$, $R^3$ et $R^4$ , comme groupes trialkylsilyles, ont de 3 à 14 atomes de carbone.

8. Composés selon la revendication 1 dans lesquels $R^5$ est l'hydrogène ou bien un alkyle en $C_1$-$C_{18}$, un cyclohexyle, un cyclopentyle un phényle ou un benzyle.

9. Composés selon la revendication 1 dans lesquels X est un radical $-NR^6R^7$, $R^6$ et $R^7$ étant chacun, indépendamment l'un de l'autre, l'hydrogène, un alkyle en $C_1$-$C_{18}$, un cyclohexyle, un cyclopentyle, un phényle ou un benzyle, ou bien $R^6$ et $R^7$ formant ensemble un groupe tétraméthylène, pentaméthylène ou 3-oxa-1,5-pentylène .

10. Composés selon la revendication 1 dont un ou deux des R1, R2, R3 et R4 sont le chlore ou le brome.

11. Composés selon la revendication 1 dans lesquels $R^2$ est le chlore ou le brome, $R^1$ et $R^4$ sont l'hydrogène et $R^3$ a l'une des significations données à la revendication 1.

12. Composés selon la revendication 1 dans lesquels $R^1$, $R^2$, $R^3$ et $R^4$ sont -F, $-CF_3$, -CN, $-NO_2$, un alcoxy ou un alkylthio en $C_1$-$C_{12}$, le groupe $Si(CH_3)_3$, un radical $-COOR^5$, $R^5$ désignant un alkyle en $C_1$-$C_{18}$, ou bien un radical -COX, X désignant un radical $-NR^6R^7$ dont $R^6$ et $R^7$ sont chacun, indépendamment l'un de l'autre, l'hydrogène, un alkyle en $C_1$-$C_{18}$, un cyclohexyle, un cyclopentyle, un phényle ou un benzyle, ou bien $R^6$ et $R^7$ forment ensemble un groupe tétraméthylène, pentaméthylène ou 3-oxa-1,5-pentylène, et de 1 à 3 des radicaux $R^1$, $R^2$, $R^3$ et $R^4$ sont l'hydrogène, le chlore ou le brome, le ou les autres ayant l'une des significations précédemment indiquées.

13. Composés selon la revendication 12 dans lesquels $R^2$, $R^3$ et $R^4$ sont -F, $-CF_3$, -CN, $-NO_2$, un alcoxy ou un alkylthio en $C_1$-$C_6$, le groupe $-Si(CH_3)_3$, ou un radical $-COOC_1$-$C_{12}$-alkyle ou $-CONR^6R^7$, $R^6$ et $R^7$ étant l'hydrogène ou un alkyle en $C_1$-$C_{12}$, et $R^1$ ou $R^1$ et $R^4$ sont l'hydrogène.

14. Procédé de préparation des composés de Formule I selon la revendication 1, procédé caractérisé en ce que l'on fait réagir un composé de Formule II

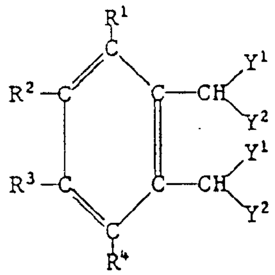

(II),

$Y^1$ et $Y^2$ désignant chacun, indépendamment l'un de l'autre, le chlore ou le brome, en présence d'acétonitrile ou d'acétone, avec de l'iodure de sodium, à une température d'au moins 40°C.

15. Procédé selon la revendication 14, caractérisé en ce que $Y^1$ et $Y^2$ sont chacun le chlore ou le brome.

16. Procédé selon la revendication 14, caractérisé en ce que l'on effectue la réaction sous reflux.

17. Procédé selon la revendication 14, caractérisé en ce que l'on opère avec au moins 1 Mol d'iodure de sodium par rapport au composé de Formule II ou avec un excès molaire pouvant s'élever jusqu'à 10 fois.

18. Procédé selon la revendication 14, caractérisé en ce que l'on utilise de l'acétonitrile comme solvant.

19. L'emploi de composés de Formule I avec un composé diénophile dans des réactions de Diels-Alder.

20. Emploi selon la revendication 19 dans lequel le composé diénophile est une benzo-1,4-quinone ou une naphto-1,4-quinone avec ou sans substituants.